# EUROPÄISCHE PATENTSCHRIFT

(11) **EP 2 482 721 B1**
(45) Veröffentlichungstag und Bekanntmachung des Hinweises auf die Patenterteilung: **28.08.2013**
(21) Anmeldenummer: 10784244.5
(22) Anmeldetag: 29.09.2010
(51) Int. Cl.: A61B 5/097, G01N 33/497

(54) **VORRICHTUNG UND VERFAHREN ZUR FRAKTIONIERTEN GEWINNUNG VON INHALTSSTOFFEN DER AUSATEMLUFT**
DEVICE AND METHOD FOR FRACTIONALLY COLLECTING CONTENTS OF EXHALED AIR
DISPOSITIF ET PROCEDE PERMETTANT D'EXTRAIRE DE MANIERE FRACTIONNEE DES COMPOSANTS DE L'AIR EXPIRE

(30) Priorität: 30.09.2009 DE 102009048644; 30.09.2009 DE 202009013577 U
(43) Veröffentlichungstag der Anmeldung: 08.08.2012
(73) Patentinhaber: MTI Medtech Innovation GmbH, 97225 Zellingen (DE)
(72) Erfinder: EICHLER, Rüdiger, 97225 Zellingen (DE)
(74) Vertreter: Kietzmann, Manfred
(86) Internationale Anmeldenummer: PCT/DE2010/001161
(87) Internationale Veröffentlichungsnummer: WO 2011/038726

(56) Entgegenhaltungen:
- WO-A1-95/31721

## Beschreibung

Die Erfindung betrifft eine Vorrichtung sowie ein Verfahren zur fraktionierten Gewinnung von Inhaltsstoffen der Ausatemluft durch Änderung deren Aggregatzustandes wie Kondensation oder Ausfrieren mittels des Joule-Thomson-Effekts, welcher bei der Expansion von unter Druck stehendem Gas auftritt.

Das Atemkondensat enthält diverse Marker und Mediatoren, welche die Zusammensetzung der bronchoalveolären, extrazellulären Auskleidungsflüssigkeit wiederspiegeln. Diese Substanzen reflektieren Veränderungen durch oxidative Schäden und Entzündungen in der Lunge sowie den Behandlungseffekt, so dass sie daher eine wichtige Rolle bei der Diagnose und Überwachung von Lungenerkrankungen spielen.

Atemkondensatsammler sind aus dem Stand der Technik bekannt.

So beschreibt die Offenlegungsschrift DE 10 2007 028 831 A1 einen Atemkondensat-Probennehmer in Form eines U-förmigen Kondensatausbildungsrohres mit einer Kühlumhüllung, die mit einer Kühlvorrichtung gekühlt wird. Das kondensierte Atemkondensat läuft in einen Kondensatabscheider und wird von dort aus in einen Kondensatsammelbehälter geleitet. Bei Erreichen einer für die Analyse ausreichenden Menge Kondensats wird der Sammelbehälter entfernt und das Kondensat wird diversen Analysemethoden zugeführt.

Aus der Offenlegungsschrift DE 197 18 925 A1 ist Atemkondensatabscheider bekannt, welcher aus einem Faservlies besteht, das einer Kühlfläche nachgeordnet oder auf einer Kühlfläche angeordnet ist. Auf der Kühlfläche kondensiert eine erste Fraktion der Inhaltsstoffe, die überwiegend aus Wasserdampf besteht, aus. Die Aerosole der Inhaltsstoffe scheiden sich als zweite Fraktion überwiegend im Faservlies ab. Die im Faservlies enthaltenen Inhaltsstoffe in Form eines Aerosols werden durch Zentrifugation, Vakuum, Druckgas oder Eluieren isoliert. Anschließend werden die Fraktionen analysiert.

Die DE 197 55 471 A1 offenbart eine Anordnung zum Auffangen von Substanzen der Ausatemluft, die eine Kühlfalle zum Abscheiden der in der Ausatemluft vorhandenen Substanzen, die in einem Winkel zum Strömungskanal angeordnet ist, und ein am Ende des Strömungskanals angeordnetem Adsorptionsgefäß aufweist.

In der EP 0 759 169 B1 ist eine Vorrichtung zum Sammeln von Atemkondensat beschrieben, welches ein von einem Kühlmantel umgebenes Probensammelrohr mit einem am Ende des Probensammelrohres angebrachten Sammelgefäß aufweist, an dessen Wandung das Atemkondensat ausfriert.

Nachteilig an den im Stand der Technik benannten Atemkondensatsammlern ist, dass zur Kondensation der Inhaltsstoffe in der Ausatemluft diese an einer Kühlfläche entlang geführt wird, was bedingt, dass eine Kondensation der Inhaltsstoffe nur im Bereich dieser Kühlflächen erfolgt. Aufgrund dessen werden auch nur geringe Atemkondensatmengen abgeschieden, wodurch der Patient angehalten ist, zur Gewinnung einer für die anschließende Analyse ausreichenden Menge Atemkondensats mehrere Atemzyklen zu vollziehen.

Aufgabe der Erfindung ist es daher, eine Vorrichtung und ein Verfahren anzugeben, mit welchem die Abscheidung von Atemkondensat aus der Ausatemluft effizienter betrieben werden kann.

Gelöst wird die Aufgabe mit einer Vorrichtung zur fraktionierten Gewinnung von Inhaltsstoffen der Ausatemluft durch Änderung deren Aggregatzustandes mittels des Joule-Thomson-Effekts entstehend bei Expansion von unter Druck stehendem Gas, enthaltend einen Strömungskanal, durch den die Ausatemluft geführt wird sowie einen Druckbehälter mit mindestens einer an den Druckbehälter angeschlossenen Entspannungsdüse, enthaltend ein unter Druck stehendes Gas oder Gasgemisch. Durch die Expansion des Gases in den Strömungskanal hinein wird die Temperatur der im Strömungskanal geführten Ausatemluft aufgrund der Abkühlung des expandieren Gases (Joule-Thomson-Effekt) auf eine Temperatur herabgesenkt, die geeignet ist, dass die zu fraktionierenden Inhaltsstoffe im Ausatemluftstrom kondensieren oder in Form von gefrorenen Partikeln im Ausatemluftstrom ausgefroren werden. Ebenso können beide Aggregatzustände nebeneinander auftreten.

Die Vorrichtung enthält des Weiteren eine Sammelvorrichtung, zur Aufnahme der im Ausatemluftstrom mitgeführten flüssigen und/oder gefrorenen Partikel, wobei die Sammelvorrichtung downstream-seitig zur Entspannungsdüse eingerichtet ist.

Inhaltsstoffen der Ausatemluft sind insbesondere Marker oder Mediatoren von Atemwegserkrankungen, wie H₂O₂, Eicosanoide, wie Isoprostan, Prostaglandin, Leukotriene, Proteine und Zytokine, Produkte der Lipidoxaidation, vasoaktive Peptide und Amine, Nitrat, Nitrit, S-Nitrosothiol, Nitrotyrosin und Elektrolyte.

Das unter Druck stehende Gas oder Gasgemisch kann im Druckbehälter in verflüssigter Form vorliegen. Das Gas kann ein trockenes Gas, vorzugsweise ein Reingas, insbesondere Argon, Stickstoff, Kohlendioxyd, ein Kohlenwasserstoff, insbesondere Propan, Butan, Pentan oder Chlorethan, sein. Das Gasgemisch kann ein trockenes Gasgemisch, bevorzugt eine Mischung aus den genannten Reingasen, insbesondere aus Kohlenwasserstoffen sein. Bei Kohlenwasserstoffgemischen werden Mischungen aus Propan, Butan, Pentan und/oder Chlorethan bevorzugt.

Eine Entspannungsdüse kann auch eine Drossel sein.

Bei dem auf dem Joule-Thomson-Effekt beruhenden Entspannungskühler wird das druckbeaufschlagte Arbeitsgas mittels einer Drossel oder einer Düse entspannt und das austretende, sich aufgrund der isenthalpen Expansion abkühlende Gas zur Temperatursenkung des Ausatemluftstroms, welcher durch den Strömungskanal strömt, eingesetzt. Die Ausatemluft wird mittels dieses Effektes soweit heruntergekühlt, dass die zu fraktionierenden Inhaltsstoffe kondensieren oder ausfrieren. Der Grad der Abkühlung richtet sich dabei nach dem zu separierenden Inhaltsstoff und dessen Phasenübergangstemperatur. Entsprechendes gilt natürlich auch für Gemische.

Durch die Entspannung das unter Druck stehenden Gases oder Gasgemisches in den Ausatemluftstrom hinein wird erreicht, dass der gesamte Ausatemluftstrom über dessen gesamten Querschnitt gleichmäßig abgekühlt wird. Das bedingt, dass auch die Kondensation und/oder das Ausfrieren der Inhaltsstoffe gleichmäßig über den gesamten Querschnitt des Ausatemluftstroms erfolgen. Im Unterschied zum Stand der Technik erfolgt somit die Kondensation oder das Ausfrieren der zu fraktionierenden Inhaltsstoffe direkt im Ausatemluftstrom und nicht an oder auf einer gekühlten Fläche. Ein weiterer Unterschied zum Stand der Technik ist, dass die flüssigen oder gefrorenen Partikel nach der Aggregatszustandsänderung im Ausatemluftstrom weiter mitgeführt werden.

Hierin liegt ein wesentlicher Vorteil gegenüber denen aus dem Stand der Technik bekannten Kondensatabscheidern, bei denen eine Kondensation nur in dem Teilvolumenstrom erfolgt, der mit der Kühlfläche in Kontakt kommt. Ein großer Anteil der Inhaltsstoffe wird beim Stand der Technik mit dem restlichen, ungekühlten Atemluftstrom in die Umgebungsluft abgegeben. Um einen repräsentativen Mittelwert der Zusammensetzung und Menge der einzelnen Inhaltsstoffe zu erhalten, ist es beim Stand der Technik daher notwendig Kondensate mehrere Atemzüge zu sammeln und für die Analyse zu vereinigen.

Diese Nachteile werden mit der vorliegenden Erfindung überwunden. Indem der gesamte Ausatemluftstrom abgekühlt wird, kann die separierbare Menge flüssigen oder gefrorenen Atemkondensats pro Volumeneinheit der Ausatmluft gegenüber dem bekannten Stand der Technik erhöht werden. Folglich sind weniger Atemzyklen oder bestenfalls nur ein Atemzug notwendig, um einerseits eine den Zustand der Lunge repräsentieren Menge und andererseits eine für die Analyse ausreichende Menge Atemkondensats abzuscheiden.

Hieraus resultiert, dass die Messwertgenauigkeit erhöht und die Zustandsbestimmung der Atemwege verbessert wird.

Mit der Effizienzsteigerung der Kondensatabscheidung geht zudem einher, dass der Patient weniger häufig in den Kondensatabscheider ausatmen muss, was angesichts der Einschränkung aufgrund seiner Atemwegserkrankung eine Entlastung für den Patienten darstellt.

Der Strömungskanal weist insbesondere eine Geometrie auf, durch die im Strömungskanal Strömungsbedingungen induzierbar sind, aufgrund derer eine Separierung der im Ausatemluftstrom mitgeführten flüssigen und/oder gefrorenen Partikel in der Sammelvorrichtung erfolgt. Der Begriff "Geometrie des Strömungskanals" umfasst die konstruktionstechnischen Parameter und Dimensionen der Bauteile der Vorrichtung, die das Strömungsverhalten der Ausatemluft sowie des flüssigen oder festen Atemkondensats im Strömungskanal bedingen. Hierunter fallen Parameter wie der Durchmesser und Länge des Strömungskanals sowie die Anzahl, Position und Krümmungsradien von Bögen oder Krümmungen.

Einfluss auf die Strömung haben ebenfalls

Querschnittsänderungen wie Verengungen oder Erweiterungen. Alle diese Parameter bestimmen das Strömungsverhalten der im Strömungskanal geführten Ausatemluft bzw. dem gegenüber der Gasphase massenträgeren flüssigen oder festen Atemkondensat.

Bei der erfinderischen Vorrichtung ist nun vorgesehen, mittels der durch die Geometrie vorgegebenen Strömungsbedingungen unter Ausnützung des Trägheitsprinzips nach der Kondensation oder dem Ausfrieren des Atemkondensats die Tröpfchen oder Eispartikel aufgrund deren Massenträgheit aus der gasförmigen Phase abzuscheiden. Dazu ist die Sammelvorrichtung im Strömungskanal an einer Stelle eingerichtet, an der aufgrund von Flieh- und/oder Schwerkräften die flüssigen und/oder festen Bestandteile aus dem mehrphasigen Strom herausgetragen werden.

Eine einfache Form einer Sammelvorrichtung ist eine Prallplatte. Bei einer Prallplatte wird der mit Flüssigkeitstropfen oder Eispartikeln beladene Ausatemluftstrom so gegen eine Platte geführt, dass der Ausatemluftstrom die Richtung ändert. Durch die Massenträgheit behalten die in dem Ausatemluftstrom enthaltene flüssigen oder gefrorenen Partikel ihre Richtung bei, treffen auf die Platte auf und werden von dort, vorzugsweise direkt auf den Sensor, abgeleitet. Eine andere Art der Trägheitsabscheidung macht sich Zentrifugalkräfte zu Nutzen. Hierbei wird der Ausatemluftstrom auf einer gekrümmten Bahn geführt. Durch die Zentrifugalkräfte werden die Tropfen oder Eispartikel auf einer äußeren Bahn, mit möglichst großem Krümmungsradius, geführt. Dadurch werden die Tropfen oder Eispartikel in diesem äußeren Bereich konzentriert. Die Tropfen oder Eispartikel können dann z.B. an einer Gleitfläche entlang des äußeren Bereichs des Ausatemluftstroms abgeschieden werden.

In einer bevorzugten Ausführungsform weist der Strömungskanal eine Krümmung auf, die vorzugsweise einen Krümmungsradius von 45 bis 90 Grad aufweist. Die einfachste Konstruktion sieht eine Krümmung von 90° vor, durch die eine Umlenkung des Ausatemluftstroms um 90° erzeugt wird, wobei dann die Sammelvorrichtung an der Stelle der Strömungskanalwandung eingerichtet ist, an der die aus der Ausatemluftstrom herausgetragenen Partikel auf die Wandung des Strömungskanals treffen würden.

Insbesondere ist die Sammelvorrichtung in Strömungsrichtung gegenüber der Einströmöffnung im Strömungskanal eingerichtet. Hierbei erfolgt die Umlenkung des Ausatemluftstroms direkt vor der Sammelvorrichtung, vorzugsweise um 90°. Die Geometrie des Strömungskanals kann dabei vorsehen, dass der Ausatemluftstrom vor der Sammelvorrichtung geteilt und eine Umlenkung eines Ausatemluftteilstroms jeweils um 90°, vorzugsweise in entgegengesetzter Richtung zum anderen Ausatemluftteilstrom, erfolgt.

Auch kann die Sammelvorrichtung direkt in der Krümmung des Strömungskanals eingerichtet sein.

Die Sammelvorrichtung kann als Drainageelement ausgebildet sein. Bei einem Drainageelement wird ein mit

Flüssigkeitstropfen beladener Gasstrom durch eine netzartige und/oder poröse Drainagestruktur geleitet. Als

Drainagestruktur kann z.B. ein Drahtgestrick oder ein Vlies, z.B. aus Kunststoff oder Glasfaser, dienen. Das Atemkondensat als Tropfen oder Eispartikel durchströmt die Drainagestruktur langsamer als der Ausatemluftstrom. Durch die Schwerkraft bewegen sich die Tropfen zum geodätisch unteren Bereich der Drainagestruktur, sammeln sich und können auf den Sensor abgeleitet werden.

Die Sammelvorrichtung kann in einer Ebene mit einem Winkel zwischen 0° und 90° zur Horizontalebene, insbesondere horizontal oder vertikal dazu, ausgerichtet sein. Der eingerichtete Winkel der Sammelvorrichtung zur Horizontalebene (Winkelebene) ergibt sich aus den durch die Geometrie des Strömungskanals induzierten Strömungsverhalten des flüssigen oder gefrorenen Atemkondensats. Je nach Strömungsverhalten der zu separierenden Partikel kann über die Winkelebene der Sammelvorrichtung der Einfallswinkel der Partikel auf die Sammelvorrichtung bestimmt werden. Bevorzugt wird ein Einfallswinkel von 90°, d.h. die Partikel treffen senkrecht auf der Sammelvorrichtung auf. Vorzugsweise ist der Winkel zur Horizontalen einstellbar (manuell oder elektronisch) und kann so dem Strömungsverhalten der Partikel angepasst werden.

In einer ersten Ausführung kann für die Gewährleistung einer gleichmäßigen Abkühlung des Ausatemluftstroms die Entspannungsdüse direkt im Strömungskanal eingerichtet sein. Hierbei wird die Expansionsdüse von der Ausatemluft umströmt. Die Expansion des unter Druck stehenden Gases erfolgt vorzugsweise in der Längsmittelachse des Strömungskanals, so dass eine Abkühlung des Ausatemluftstroms ausgehend von der Längsmittelachse in Richtung der Wandung erfolgt.

Bei einer zweiten Ausführung kann die Entspannungsdüse in der Wand des Strömungskanals, vorzugsweise als Ringdüse, eingerichtet sein. Hierbei wird der Ausatemstrom ausgehend vom Randbereich in Richtung der Längsmittelachse des Ausatemluftstroms abgekühlt.

Die Entspannungsdüse und oder Entspannungsdüsen können bei beiden Alternativen in Strömungsrichtung, quer zur Strömungsrichtung und/oder gegen die Strömungsrichtung ausgerichtet sein. Je nach Ausrichtung der Entspannungsdüse wird die Expansion des Gases zur Beschleunigung oder Reduzierung des Flows beitragen sowie zur Verwirbelung des Ausatemluftstroms und damit zur Ausbildung einer turbulenten Strömung führen.

In einer besonderen Ausführungsform ist ein regel- und steuerbarer Durchflussregler eingerichtet, mit dem der Durchfluss durch die Entspannungsdüse des expandierenden Gases oder Gasgemisches einstellbar und an die Strömungsgeschwindigkeit der Ausatemluft im Strömungskanal anpassbar ist. Hierdurch kann der Grad der Verwirbelung des Ausatemluftstroms sowie dessen Strömungsgeschwindigkeit als auch der Prozess der Kondensation bzw. der Eisbildung maßgeblich beeinflusst und bestimmt werden. Entsprechend kann auf die Varianzen der Ausatmung des Patienten, der nicht gleichmäßig ausatmet, reagiert werden. Ebenso kann mittels des regelbaren und steuerbaren Durchflussreglers auf Temperaturvarianzen verschiedener Ausatemluftströme reagiert werden. Es versteht sich, dass die Vorrichtung zur Steuerung und Regelung der Bauteile über eine zentrale Steuer- und Regeleinheit, einen Prozessor, verfügt, welche die Systemdaten verarbeitet und kontrolliert.

Vorzugsweise ist zusätzlich in der Wandung des Strömungskanals oder im Strömungskanal direkt mindestens ein Temperatursensor zum Messen der Temperatur des Ausatemluftstroms eingerichtet, der mit dem Prozessor verbunden ist. Der von dem Temperatursensor ermittelte Temperaturwert des Ausatemluftstroms wird an den Prozessor weitergeleitet. Auch kann die Temperatur mittels eines berührungslosen IR-Temperatursensors (Infrarot-Temperatursensor) gemessen werden. Der Prozessor ermittelt anhand des ermittelten Temperaturwertes diejenigen Betriebseinstellungen des Durchflussreglers, mit welchen der Ausatemluftstrom durch Expansion des unter Druck stehenden Gases unterhalb der Taupunktslinie oder den Gefrierpunkt der Gaszusammensetzung des Ausatemluftstroms abgekühlt werden kann.

In einer weiteren Ausführungsform verfügt die Vorrichtung über eine temperierbare Sammelvorrichtung, wobei die Sammelvorrichtung ein Temperierelement aufweist, mit dem die Temperatur der Sammelvorrichtung einstellbar, steuerbar und/oder regelbar ist. Mittels des Temperierelements wird die Sammelvorrichtung beheizt oder gekühlt, je nach Verfahrensschritt oder Bedingungen im Strömungskanal. Durch eine Kühlung der Sammelvorrichtung auf eine Temperatur beispielsweise unterhalb des Gefrierpunks während der Separierung wird verhindert, dass abgeschiedenes flüssiges oder gefrorenes Atemkondensat von dem Ausatemluftstrom mitgerissen wird, da es an der Sammeleinrichtung festfriert. Ebenfalls wird verhindert, dass das gefrorene Atemkondensat wieder auftaut und in die Gasphase übergeht.

Nach der Separierung kann die Sammelvorrichtung aufgeheizt werden, um das separierte Atemkondensat aufzutauen oder weiterreichend, auf Analysetemperatur zu erwärmen. Ein Erwärmen auf Analysetemperatur ist vorgesehen, wenn die Sammeleinrichtung direkt mit einem Sensor verbunden ist oder die Sensormessfläche selbst darstellt.

Die Sammelvorrichtung kann direkt im Strömungskanal oder in oder an der Wandung des Strömungskanals eingerichtet sein. Vorzugsweise ist diese lösbar mit der Vorrichtung verbunden, so dass diese herausgenommen werden kann. Das in der Sammelvorrichtung separierte Atemkondensat kann dann externen Analysemethoden bereitgestellt werden. Da vorgesehen ist, dass die Sammeleinrichtung gleichzeitig zur Analytik eingesetzt werden kann, können so in der Vorrichtung diverse modifizierte Sammeleinrichtungen verwendet werden. Beispielsweise sind hier Modifikationen in chemischer, biochemischer oder physikalischchemischer Form vorgesehen, wie Beschichtungen, die nur bestimmte Inhaltsstoffe binden oder umsetzen, enzymatische Beschichtungen oder auch ad- oder absorptive Beschichtungen, Reaktionslösungen oder andere die Inhaltsstoffe bindende Verbindungen oder Substanzen. Die Aufzählung ist nicht begrenzend, sondern schließt alle Modifikationen, die im Bereich der Nachweisanalytik verwendet werden, ein. Insbesondere ist vorgesehen, die Sammelvorrichtung derart zu modifizieren, dass die Leitfähigkeit des Atemkondensats durch Aufnahme von Salzen erhöht und für die Bestimmung mittels der entsprechenden Sensoren eingestellt wird.

Bevorzugt stellt die Sammelvorrichtung die Messfläche eines Sensors dar. Damit kann das Atemkondensat direkt nach dessen Separierung analysiert werden, ohne dass es der Vorrichtung entnommen werden muss.

Auch kann die Sammelvorrichtung mit einem Sensor derart verbunden sein, dass die fraktionierten Inhaltsstoffe in flüssiger und/oder gefrorener Form auf die Messfläche des Sensors leitbar sind. Konstruktive Maßnahme stellen hierbei Leitungen oder insbesondere Gleitflächen dar, mit denen das Atemkondensat auf die Sensoroberfläche geführt wird.

Bei einer verbesserten Konstruktion ist die Sammelvorrichtung direkt auf der Messfläche des Sensors eingerichtet. Vorzugsweise ist die Sammelvorrichtung mit einer die zu fraktionierenden Inhaltsstoffe speichernden Schicht, beispielsweise mit einem saugenden oder einer absorbierenden Schicht, ausgestattet ist. Mögliche Speichermedien sind einfache Gewebe, Vliese, Schäume, aber auch Superabsorber oder Gele.

In der Spirometrie werden regelmäßig Pneumotachographen eingesetzt. Zwischenzeitlich sind auch Pneumotachographen im Handel erhältlich, mit welchen die Inhaltsstoffe der Ausatemluft analysiert werden. Es ist vorgesehen, dass die erfindungsgemäße Vorrichtung in Verbindung mit einem Pneumotachographen oder einem anderen Lungenfunktionsanalysegerät eingesetzt wird. Hierzu weist die erfindungsgemäße Vorrichtung an der eingangsseitigen Öffnung Verbindungsmittel zum Verbinden mit der Auslassöffnung des entsprechenden Gerätes aus dem Bereich der Lungenfunktionsdiagnostik auf. In der einfachsten Form ist dies ein Steckverbindung in Form eines offenen Rohrendes, welches auf die Auslassöffnung aufsteckbar oder in diese einsteckbar ist. Die Steckverbindung kann konisch oder zylindrisch und mit O-Ring-Dichtungen ausgestattet sein. Ebenfalls sind Gewindeanschlüsse, Bajonettverschlüsse oder andere lösbare Verbindungen wie Klemm- oder Klickverbindungen vorgesehen.

Um u.a. ein Abscheiden des flüssigen oder festen Atemkondensats an der Wandung des Strömungskanals zu verhindern, kann auf oder in der Wandung des Strömungskanals eine temperierbare Schicht eingerichtet sein, mit welcher die Wandung des Strömungskanals beheizt oder gekühlt werden kann. Ebenso kann mittels der temperierbaren Schicht eine Temperierung des Strömungskanals erfolgen.

Darüber hinaus beansprucht die Erfindung ein Verfahren zur fraktionierten Gewinnung von Inhaltsstoffen der Ausatemluft durch Änderung deren Aggregatzustandes mittels des Joule-Thomson-Effekts entstehend bei Expansion von unter Druck stehendem Gas. Hierbei wird durch Expansion eines Gases oder eines Gasgemisches in einen Strömungskanal hinein die Temperatur der im Strömungskanal geführten Ausatemluft auf eine Temperatur herabgesenkt, bei der die zu fraktionierenden Inhaltsstoffe im Ausatemluftstrom kondensieren und/oder in Form von gefrorenen Partikeln im Ausatemluftstrom auszufrieren. Nach dem Ausfrieren der zu fraktionierenden Inhaltstoffe werden diese als flüssige und/oder gefrorene Partikel im Ausatemluftstrom mitgeführt und aufgrund der Geometrie des Strömungskanals und der daraus resultierenden Strömungsbedingungen im Strömungskanal in einer Sammelvorrichtung separiert.

Das Verfahren kann zusätzlich den Verfahrensschritt aufweisen, dass während der Fraktionierung der Inhaltsstoffe durch deren Kondensation und/oder Ausfrieren im Ausatemluftstrom und deren Separierung in oder auf der Sammelvorrichtung eine Kühlung der Sammelvorrichtung auf ≤ 0°C erfolgt.

Zusätzlich kann der Verfahrensschritt vorgesehen sein, dass während der Fraktionierung der zu separierenden Inhaltsstoffe durch deren Kondensation und/oder Ausfrieren im Ausatemluftstrom und deren Separierung in oder auf der Sammelvorrichtung eine Temperierung, vorzugsweise ein Beheizen, der in der Wandung des Strömungskanals eingerichteten temperierbaren Schicht erfolgt.

Vorzugsweise umfasst das erfindungsgemäße Verfahren den Verfahrensschritt, bei dem nach der Separierung der im Ausatemluftstrom mitgeführten gefrorenen Partikel zum Schmelzen der gefrorenen Partikel in der Sammelvorrichtung die Sammelvorrichtung auf eine Temperatur > 0°C, vorzugsweise auf die sensorspezifische Betriebstemperatur des Sensors, insbesondere auf eine Temperatur von 10°C bis 40°C, aufgeheizt wird.

Der Begriff "sensorspezifische Betriebstemperatur" beschreibt den Temperaturbereich, in welchem der Sensor im Messbetrieb betrieben wird. Die Temperatur ist abhängig von der Art und der Funktionsweise des verwendeten Sensors. Die Erfindung sieht den Einsatz aller üblichen, in der Probenanalytik einsetzbaren Sensoren vor.

Auch kann das Verfahren den Verfahrensschritt umfassen, dass die Dauer der Gasexpansion, das Expansionsvolumen und/oder die durch die Expansion des Gases oder Gasgemisches bewirkte Temperatur des Ausatemluftstroms gesteuert und/oder geregelt wird, wobei vorzugsweise das Maß und die Dauer der Expansion vom Flow und/oder der Temperatur des Ausatemluftstroms und/oder einer definierten Fraktion des Ausatemluftstroms abhängig sind.

Das Verfahren sieht in einer besonderen Ausführungsform zusätzlich den Verfahrensschritt vor, dass die fraktionierten und separierten Inhaltsstoffe qualitativ und/oder quantitativ bestimmt werden. Dies kann mittels des vorrichtungsseitigen Sensors oder mittels externer Analytik erfolgen.

Nachstehend wird die Erfindung anhand von drei Ausführungsformen näher erläutert.

Die Figuren zeigen
- Fig.1:: eine erste Ausführungsform mit einer Teilung und Umlenkung des Ausatemluftstroms in zwei entgegengesetzte Richtungen
- Fig. 2:: eine zweite Ausführungsform mit einer Umlenkung des Ausatemluftstroms um 90° und einer Separierung mittels Schwerkraft
- Fig.3:: eine dritte Ausführungsform mit einer Umlenkung des Ausatemluftstroms um ca. 45° und einer Separierung mittels Schwerkraft und Fliehkraft

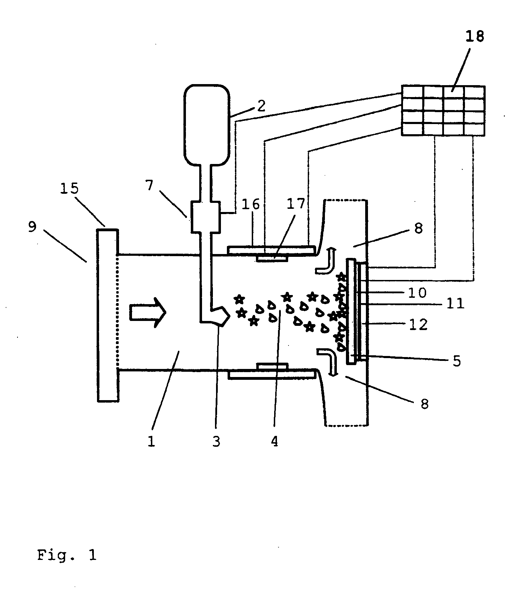

Die Figur 1 zeigt eine erste Ausführungsform der Vorrichtung zur fraktionierten Gewinnung von Inhaltsstoffen der Ausatemluft. Die Ausatemluft des Patienten wird durch die eingangsseitige Öffnung 9 in den Strömungskanal 1 geführt, in welchem die Ausatemluft weitergeleitet wird. Die eingangsseitige Öffnung 9 ist als geweitetes Rohrende als Mittel zum Verbinden 14 ausgeführt, welches auf die ausgangsseitige Öffnung eines Gerätes der Lungenfunktionsdiagnostik (nicht dargestellt) aufgesteckt werden kann. Im Strömungskanal 1 ist in dessen Längsachse eine Entspannungsdüse 3 eingerichtet, die vom Ausatemluftstrom umströmt wird. Die Entspannungsdüse ist über eine Leitung mit einem Druckbehälter 2, welcher ein unter Druck stehendes trockenes Gas oder trockenes Gasgemisch enthält, verbunden. In der Leitung zwischen Entspannungsdüse 3 und Druckbehälter 2 ist ein Durchflussregler 7 eingerichtet, mit welchem der Durchfluss des expandierenden Gases oder Gasgemisches durch die Entspannungsdüse 3 einstellbar und an die Strömungsgeschwindigkeit der Ausatemluft im Strömungskanal 1 anpassbar ist. Während der Ausatemluftstrom (Pfeil) die Entspannungsdüse 3 umströmt, wird diese geöffnet und das im Druckbehälter 2 enthaltene Gas entspannt sich über die Entspannungsdüse 3 in den Strömungskanal hinein. Dies bewirkt eine Abkühlung des Ausatemluftstroms aufgrund des Joule-Thomson-Effekts, welcher bei der Expansion von unter Druck stehendem Gas entsteht.

Durch die Abkühlung des Ausatemluftstroms auf eine Temperatur, die mindestens unterhalb der Taupunktslinie des feuchten Ausatemluftstroms liegt, kondensiert das Atemkondensat im Ausatemluftstrom in Form von Tropfen oder Nebel. Alternativ kann die Temperatur bis unterhalb des Gefrierpunktes des Ausatemluftstroms abgesenkt werden. Hierdurch wird erreicht, dass das Atemkondensat im Ausatemluftstrom ausfriert. Beide Aggregatzustände können in Abhängigkeit von der Temperaturabsenkung auch nebeneinander im Ausatemluftstrom vorliegen.

Nach der Aggregatszustandsänderung des Atemkondensats wird dieses als Nebel, Tropfen oder in Form von Eispartikeln oder - kristallen 4 im Ausatemluftstrom weiter mitgeführt.

Die Geometrie des Strömungskanals 1 ist T-förmig ausgestaltet und weist zwei Auslassöffnungen auf, die gegenüber liegen. Der Krümmungsradius 8 des Strömungskanals 1 im T-Bereich beträgt jeweils 90°.

Die Wandung des Strömungskanals 1 zwischen Entspannungsdüse 3 und Krümmung 8 ist mit einer temperierbaren Schicht 16 ausgestattet, welche sowohl zur Kühlung als auch zum Beheizen der Wandung und des Strömungskanals 1 eingerichtet ist.

Die Sammelvorrichtung 5 zur Aufnahme der im Ausatemluftstrom mitgeführten flüssigen und/oder gefrorenen Partikel 4 ist downstream-seitig zur Entspannungsdüse 3 gegenüber der eingangseitigen Öffnung 9 in geradliniger Verlängerung des eingangseitigen Bereichs des Strömungskanals an der Wandung eingerichtet. Direkt vor der Sammelvorrichtung 5 wird Ausatemluftstrom geteilt und jeweils um 90° aufgrund der Krümmung des Strömungskanals 1 umgelenkt.

Durch die Massenträgheit der flüssigen und/oder gefrorenen Partikel 4 behalten diese in dem Ausatemluftstrom trotz dessen Umlenkung um 90° nahezu ihre geradlinige Strömungsrichtung bei und treffen auf die Sammelvorrichtung 5 auf, wo sie sich abscheiden. Die Sammelvorrichtung 5 ist vertikal eingerichtet. Die Sammelvorrichtung 5 ist mit einem Temperierelement 10 ausgestattet, welches zum Kühlen, oder Erwärmen der Sammelvorrichtung eingerichtet ist. Hinter der Sammelvorrichtung 5 ist der Sensor 12 eingerichtet. Die Messfläche 11 des Sensors 12 schließt vollflächig mit der Sammelvorrichtung 5 ab, so dass die flüssigen und/oder gefrorenen Partikel 4 direkt von der Sammelvorrichtung 5 auf de Messfläche 11 des Sensors 12 geleitet und dort qualitativ und quantitativ bestimmt werden können.

In der Wandung des Strömungskanals 1 ist ein berührloser Temperatursensor 17 zum Messen der Temperatur des Ausatemluftstroms eingerichtet, der mit einem Prozessor 18 verbunden ist. Die von dem Temperatursensor 17 ermittelte Temperatur des Ausatemluftstroms wird in Form eines Messsignals an den Prozessor 18 weitergeleitet (Punkt-Strich-Linie) und dort verarbeitet. Der Prozessor 18 generiert anhand des Messsignals u.a. entsprechende Steuersignale zur Steuerung und Regelung des Durchflussreglers 7, zur Steuerung und Regelung des Temperierelements 10, zur Steuerung und Regelung des Sensors 12 und/oder dessen Messfläche 11 und/oder zur Steuerung und Regelung der temperierbaren Schicht 16.

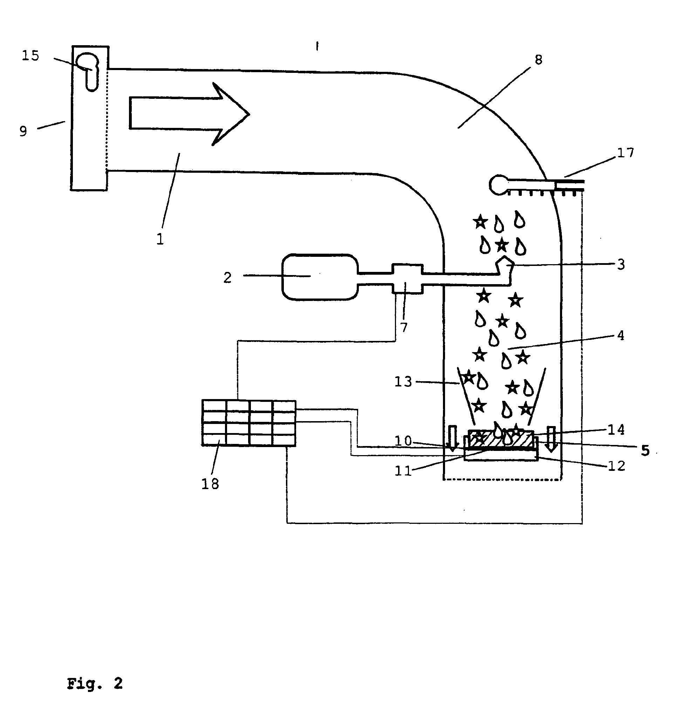

Die Figur 2 zeigt eine zweite Ausführungsform der Vorrichtung. Die Ausatemluft des Patienten wird durch die eingangsseitige Öffnung 9 in den Strömungskanal 1 geführt, in welchem die Ausatemluft weitergeleitet wird. Die eingangsseitige Öffnung 9 ist mit einem Bajonettverschluss als Mittel zum Verbinden 14 ausgeführt, welches auf die ausgangsseitige Öffnung eines Gerätes der Lungenfunktionsdiagnostik (nicht dargestellt) aufgedreht werden kann.

Die Geometrie des Strömungskanals 1 sieht eine Krümmung 8 des Strömungskanals mit einem Krümmungsradius von 90° vor. Hierdurch wird die horizontale Strömungsrichtung des Ausatemluftstroms in eine vertikale Strömungsrichtung umgelenkt.

Im Strömungskanal 1 ist nach der Krümmung 8 in dessen Längsachse eine Entspannungsdüse 3 eingerichtet, die vom Ausatemluftstrom umströmt wird. Die Entspannungsdüse ist entgegen der Strömungsrichtung ausgerichtet und über eine Leitung mit einem Druckbehälter 2, welcher ein unter Druck stehendes trockenes Gas oder trockenes Gasgemisch enthält, verbunden. In der Leitung zwischen Entspannungsdüse 3 und Druckbehälter 2 ist ein Durchflussregler 7 eingerichtet, mit welchem der Durchfluss des expandierenden Gases oder Gasgemisches durch die Entspannungsdüse 3 einstellbar und an die Strömungsgeschwindigkeit der Ausatemluft im Strömungskanal 1 anpassbar ist.

Die Sammelvorrichtung 5 zur Aufnahme der im Ausatemluftstrom mitgeführten flüssigen und/oder gefrorenen Partikel 4 ist downstream-seitig zur Entspannungsdüse 3 in geradliniger Verlängerung des ausgangsseitigen Bereichs des Strömungskanals im Strömungskanal eingerichtet. Die Sammelvorrichtung 5 ist horizontal ausgerichtet.

Während der Ausatemluftstrom (Pfeil) die Entspannungsdüse 3 umströmt, wird diese geöffnet und das im Druckbehälter 2 enthaltene Gas entspannt sich über die Entspannungsdüse 3 in den Strömungskanal hinein. Dies bewirkt eine Abkühlung des Ausatemluftstroms aufgrund des Joule-Thomson-Effekts, welcher bei der Expansion von unter Druck stehendem Gas entsteht. Durch die Abkühlung des Ausatemluftstroms kondensiert das im Ausatemluftstrom enthaltende Atemkondensat in Form von Nebel oder Tröpfchen und/oder friert in Form von Eiskristallen oder Eispartikeln aus, je nach Grad der Abkühlung des Ausatemluftstroms.

Die flüssigen und/oder gefrorenen Partikel 4 werden anschließend im gasförmigen Anteil des Ausatemluftstroms weiter mitgeführt. Die Abscheidung der flüssigen und/oder gefrorenen Partikel 4 erfolgt aufgrund ihrer Massenträgheit und der Schwerkraft in der Sammelvorrichtung 5.

Der gasförmige Anteil des Ausatemluftstroms wird wandungsseitig an der Sammelvorrichtung 5 vorbeigelenkt.

Die Sammelvorrichtung 5 ist mit Gleitflächen 13 ausgestattet, mit denen die fraktionierten Inhaltsstoffe 4 in flüssiger und/oder gefrorener Form auf die Messfläche 11 des Sensors 12 leitbar sind. Die Gleitflächen 13 können beispielsweise in Form eines Trichters oder einer auf dem Kopf stehenden Pyramide ausgestaltet sein.

Die Sammelvorrichtung 5 ist mit einem Temperierelement 10 ausgestattet, welches zum Kühlen oder Erwärmen der Sammelvorrichtung eingerichtet ist. Hinter der Sammelvorrichtung 5 ist der Sensor 12 eingerichtet. Die Messfläche 11 des Sensors 12 schließt vollflächig mit der Sammelvorrichtung 5 ab, so dass die flüssigen und/oder gefrorenen Partikel 4 direkt von der Sammelvorrichtung 5 auf de Messfläche 11 des Sensors 12 geleitet und dort qualitativ und quantitativ bestimmt werden können.

Die Sammelvorrichtung 5 ist zusätzlich mit einer die zu fraktionierenden Inhaltsstoffe 4 speichernden Schicht 14 ausgestattet, welche in direktem Kontakt mit der Messfläche 11 des Sensors 12 steht.

Im Strömungskanal 1 ist ein Temperatursensor 17 zum Messen der Temperatur des Ausatemluftstroms eingerichtet, der mit einem Prozessor 18 verbunden ist. Die von dem Temperatursensor 17 ermittelte Temperatur des Ausatemluftstroms wird in Form eines Messsignals an den Prozessor 18 weitergeleitet (Punkt-Strich-Linie) und dort verarbeitet. Der Prozessor 18 generiert anhand des Messsignals u.a. entsprechende Steuersignale zur Steuerung und Regelung des Durchflussreglers 7, zur Steuerung und Regelung des Temperierelements 10 und/oder zur Steuerung und Regelung des Sensors 12 und/oder dessen Messfläche 11.

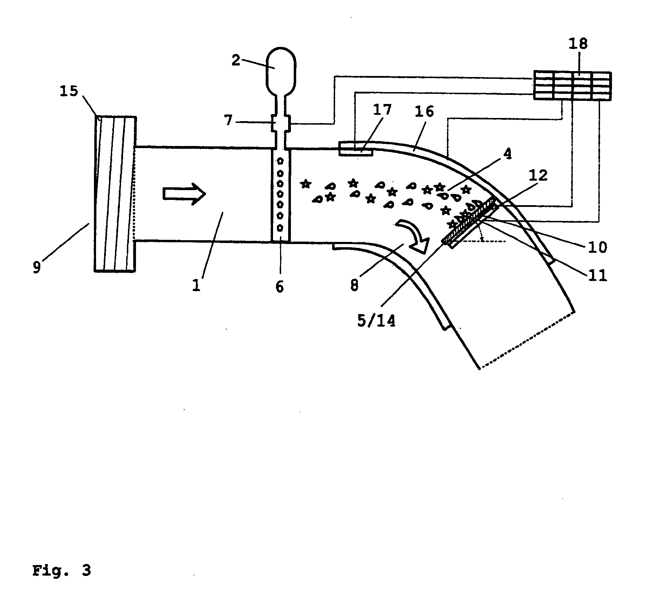

In der Figur 3 ist eine dritte Ausführungsform der Vorrichtung dargestellt.

Die Ausatemluft des Patienten wird durch die eingangsseitige Öffnung 9 in den Strömungskanal 1 geführt, in welchem die Ausatemluft weitergeleitet wird. Die eingangsseitige Öffnung 9 ist mit Gewinde als Mittel zum Verbinden 14 ausgeführt, welches auf die ausgangsseitige Öffnung eines Gerätes der Lungenfunktionsdiagnostik (nicht dargestellt) aufgedreht werden kann. Das Gewinde kann ein Innen- oder Außengewinde sein.

Die Geometrie des Strömungskanals 1 sieht eine Krümmung 8 des Strömungskanals mit einem Krümmungsradius von 45° vor.

Hierdurch wird die horizontale Strömungsrichtung des Ausatemluftstroms um 45° umgelenkt.

Im Strömungskanal 1 ist vor der Krümmung 8 in dessen Wandung eine Entspannungsdüse 3 in Form einer Ringdüse 6 eingerichtet, die vom Ausatemluftstrom durchströmt wird. Die Entspannungsdüse 3 der Ringdüse 6 ist quer zur Strömungsrichtung ausgerichtet und über eine Leitung mit einem Druckbehälter 2, welcher ein unter Druck stehendes trockenes Gas oder trockenes Gasgemisch enthält, verbunden. In der Leitung zwischen Entspannungsringdüse 6 und Druckbehälter 2 ist ein Durchflussregler 7 eingerichtet, mit welchem der Durchfluss des expandierenden Gases oder Gasgemisches durch die Entspannungsringdüse 6 einstellbar und an die Strömungsgeschwindigkeit der Ausatemluft im Strömungskanal 1 anpassbar ist.

Die Sammelvorrichtung 5 zur Aufnahme der im Ausatemluftstrom mitgeführten flüssigen und/oder gefrorenen Partikel 4 ist downstream-seitig zur Entspannungsringdüse 6 in der Krümmung 8 des Strömungskanals 1 im Strömungskanal 1 eingerichtet. Die Sammelvorrichtung 5 ist in einem Winkel zur Horizontalebene, der variabel einstellbar ist, ausgerichtet. Der Winkel der Sammelvorrichtung 5 ist einstellbar (Punkt-Strich-Linie) in einem Winkelbereich von 20° bis 70°, vorzugsweise 30° bis 60°, insbesondere 40° bis 50°.

Während der Ausatemluftstrom (Pfeil) die Entspannungsringdüse 6 durchströmt, wird diese geöffnet und das im Druckbehälter 2 enthaltene Gas entspannt sich über die Entspannungsringdüse 6 in den Strömungskanal hinein. Dies bewirkt eine Abkühlung des Ausatemluftstroms aufgrund des Joule-Thomson-Effekts, welcher bei der Expansion von unter Druck stehendem Gas entsteht. Durch die Abkühlung des Ausatemluftstroms kondensiert das im Ausatemluftstrom enthaltende Atemkondensat in Form von Nebel oder Tröpfchen und/oder friert in Form von Eiskristallen oder Eispartikeln aus, je nach Grad der Abkühlung des Ausatemluftstroms.

Die flüssigen und/oder gefrorenen Partikel 4 werden anschließend im gasförmigen Anteil des Ausatemluftstroms weiter mitgeführt. Die Abscheidung der flüssigen und/oder gefrorenen Partikel 4 erfolgt nach dem Trägheitsprinzip aufgrund ihrer Massenträgheit und der Schwerkraft in der Sammelvorrichtung 5.

Der gasförmige Anteil des Ausatemluftstroms wird wandungsseitig an der Sammelvorrichtung 5 vorbeigelenkt.

Die Sammelvorrichtung 5 ist zusätzlich mit einer die zu fraktionierenden Inhaltsstoffe 4 speichernden Schicht 14 ausgestattet, welche in direktem Kontakt mit der Messfläche 11 des Sensors 12 steht.

Die Sammelvorrichtung 5 ist mit einem Temperierelement 10 ausgestattet, welches zum Kühlen oder Erwärmen der Sammelvorrichtung eingerichtet ist. Hinter der Sammelvorrichtung 5 ist der Sensor 12 eingerichtet. Die Messfläche 11 des Sensors 12 schließt vollflächig mit der Sammelvorrichtung 5 ab, so dass die flüssigen und/oder gefrorenen Partikel 4 direkt von der Sammelvorrichtung 5 auf de Messfläche 11 des Sensors 12 geleitet und dort qualitativ und quantitativ bestimmt werden können.

In der Wandung des Strömungskanals 1 ist ein Temperatursensor 17 zum Messen der Temperatur des Ausatemluftstroms eingerichtet, der mit einem Prozessor 18 verbunden ist. Die von dem Temperatursensor 17 ermittelte Temperatur des Ausatemluftstroms wird in Form eines Messsignals an den Prozessor 18 weitergeleitet (Punkt-Strich-Linie) und dort verarbeitet. Der Prozessor 18 generiert anhand des Messsignals u.a. entsprechende Steuersignale zur Steuerung und Regelung des Durchflussreglers 7, zur Steuerung und Regelung des Temperierelements 10, zur Steuerung und Regelung des Sensors 12 und/oder dessen Messfläche 11 und/oder der temperierbaren Schicht 16.

Die Wandung des Strömungskanals 1 ist mit einer temperierbaren Schicht 16 ausgestattet, welche sowohl zur Kühlung als auch zum Beheizen der Wandung des Strömungskanals 1 und/oder der Temperierung des Ausatemluftstroms eingerichtet ist. Die temperierbare Schicht 16 befindet sich auf der Innenseite der Wandung downstream-seitig zur Entspannungsringdüse 6 und verläuft über die Krümmung 8 des Strömungskanals 1 hinaus.

### Bezugszeichenliste:

- 1: Strömungskanal
- 2: Druckbehälter
- 3: Entspannungsdüse
- 4: Kondensiertes und/oder gefrorenes Atemkondensat
- 5: Sammelvorrichtung
- 6: Ringdüse
- 7: Durchflussregler
- 8: Krümmung
- 9: eingangsseitige Öffnung
- 10: Temperierelement
- 11: Messfläche des Sensors
- 12: Sensor
- 13: Gleitfläche
- 14: Speichernde Schicht
- 15: Mittel zum Verbinden
- 16: Temperierbare Schicht
- 17: Temperatursensor
- 18: Prozessor

## Patentansprüche

1. Vorrichtung zur fraktionierten Gewinnung von Inhaltsstoffen der Ausatemluft durch Änderung deren Aggregatzustandes mittels des Joule-Thomson-Effekts entstehend bei Expansion von unter Druck stehendem Gas, enthaltend
einen Strömungskanal (1), durch den die Ausatemluft führbar ist,
einen Druckbehälter (2) mit mindestens einer an den Druckbehälter (2) angeschlossenen Entspannungsdüse (3), enthaltend ein unter Druck stehendes Gas oder Gasgemisch, mit welchem durch dessen Expansion in den Strömungskanal (1) hinein die Temperatur der im Strömungskanal (1) geführten Ausatemluft auf eine Temperatur herabsenkbar ist, die geeignet ist, die zu fraktionierenden Inhaltsstoffe im Ausatemluftstrom zu kondensieren und/oder in Form von gefrorenen Partikeln (4) im Ausatemluftstrom auszufrieren,
eine Sammelvorrichtung (5) zur Aufnahme der im Ausatemluftstrom mitgeführten flüssigen und/oder gefrorenen Partikel (4), wobei die Sammelvorrichtung (5) downstream-seitig zur Entspannungsdüse (3) eingerichtet ist.

2. Vorrichtung nach Anspruch 1, **dadurch gekennzeichnet, dass**
der Strömungskanal (1) eine Geometrie aufweist, mit welcher im Strömungskanal (1) Strömungsbedingungen induzierbar sind, aufgrund derer eine Separierung der im Ausatemluftstrom mitgeführten flüssigen und/oder gefrorenen Partikel (4) direkt aus dem Ausatemluftstrom in der Sammelvorrichtung (5) erfolgt.

3. Vorrichtung nach Anspruch 1 oder 2, **dadurch gekennzeichnet, dass**
mindestens eine Entspannungsdüse (3) im Strömungskanal (1), vorzugsweise in der Längsmittelachse des Strömungskanals (1), eingerichtet ist.

4. Vorrichtung nach Anspruch 1 oder 2, **dadurch gekennzeichnet, dass**
mindestens eine Entspannungsdüse (3) in der Wand des Strömungskanals (1), vorzugsweise als Ringdüse (6), eingerichtet ist.

5. Vorrichtung nach einem der Ansprüche 1 bis 4, **dadurch gekennzeichnet, dass**
ein regel- und steuerbarer Durchflussregler (7), mit dem der Durchfluss des expandierenden Gases oder Gasgemisches durch die Entspannungsdüse (3) einstellbar und an die Strömungsgeschwindigkeit der Ausatemluft im Strömungskanal (1) anpassbar ist, eingerichtet ist.

6. Vorrichtung nach einem der Ansprüche 1 bis 5, **dadurch gekennzeichnet, dass**
der Strömungskanal (1) eine Krümmung (8), vorzugsweise von 45 bis 90 Grad, aufweist.

7. Vorrichtung nach Anspruch 6, **dadurch gekennzeichnet, dass**
die Sammelvorrichtung (5) direkt in der Krümmung (8) des Strömungskanals (1) eingerichtet ist.

8. Vorrichtung nach einem der Ansprüche 1 bis 7, **dadurch gekennzeichnet, dass**
die Sammelvorrichtung (5) in Strömungsrichtung gegenüber der eingangsseitigen Öffnung (9) im Strömungskanal (1) eingerichtet ist.

9. Vorrichtung nach einem der Ansprüche 1 bis 8, **dadurch gekennzeichnet, dass**
die Sammelvorrichtung (5) ein Temperierelement (10) aufweist, mit dem die Temperatur der Sammelvorrichtung (5) einstellbar, steuerbar und/oder regelbar ist.

10. Vorrichtung nach einem der Ansprüche 1 bis 9, **dadurch gekennzeichnet, dass**
die Sammelvorrichtung (5) mit Leitungen und/oder Gleitflächen (13) ausgestattet ist, mit denen die flüssigen und/oder gefrorenen Partikel (4) in oder auf die Sammelvorrichtung (5) leitbar sind.

11. Vorrichtung nach einem der Ansprüche 1 bis 10, **dadurch gekennzeichnet, dass**
auf oder in der Wandung des Strömungskanals (1) eine temperierbare Schicht (16) eingerichtet ist.

12. Vorrichtung nach einem der Ansprüche 1 bis 11, **dadurch gekennzeichnet, dass**
in und/oder an der Wandung des Strömungskanals (1) und/oder im Strömungskanal (1) direkt mindestens ein Temperatursensor (17) zum Messen der Temperatur des Ausatemluftstroms eingerichtet ist.

13. Verfahren zur fraktionierten Gewinnung von Inhaltsstoffen der Ausatemluft durch Änderung des Aggregatzustandes der Inhaltsstoffe mittels des Joule-Thomson-Effekts,
bei dem ein in einem Strömungskanal (1) geführter Ausatemluftstrom durch Entspannung eines in einem Druckbehälter (2) enthaltenen und unter Druck stehenden Gases oder Gasgemisches in den im Strömungskanal (1) geführten Ausatemluftstrom hinein auf eine Temperatur abgekühlt wird, bei der die zu fraktionierenden Inhaltsstoffe der Ausatemluft im Ausatemluftstrom zu flüssigen Partikeln (4) kondensieren und/oder in Form von gefrorenen Partikeln (4) ausfrieren,
die als flüssige und/oder gefrorene Partikel (4) fraktionierten Inhaltstoffe im Ausatemluftstrom mitgeführt und in einer Sammelvorrichtung (5) separiert werden.

14. Verfahren nach Anspruch 13, **dadurch gekennzeichnet, dass**
durch die Geometrie des Strömungskanals (1) Strömungsbedingungen im Strömungskanal (1) induziert werden, welche dazu führen, dass die flüssigen und/oder gefrorenen Partikel (4) direkt in der Sammelvorrichtung (5) separiert werden.

15. Verfahren nach einem der Ansprüche 13 oder 14, **dadurch gekennzeichnet, dass**
während der Fraktionierung der Inhaltsstoffe durch deren Kondensation und/oder Ausfrieren im Ausatemluftstrom und deren Separierung in oder auf der Sammelvorrichtung (5) eine Kühlung der Sammelvorrichtung (5) auf ≤ 0°C erfolgt.

16. Verfahren nach einem der Ansprüche 13 bis 15, **dadurch gekennzeichnet, dass**
während der Fraktionierung der Inhaltsstoffe durch deren Kondensation und/oder Ausfrieren im Ausatemluftstrom und deren Separierung in oder auf der Sammelvorrichtung (5) eine Temperierung, vorzugsweise ein Beheizen, einer in der Wandung des Strömungskanals (1) eingerichteten temperierbaren Schicht (16) erfolgt.

17. Verfahren nach einem der Ansprüche 13 bis 16, **dadurch gekennzeichnet, dass**
die Dauer der Gasexpansion, das Expansionsvolumen und/oder die durch die Expansion des Gases oder Gasgemisches bewirkte Temperatur des Ausatemluftstroms gesteuert und/oder geregelt wird, wobei vorzugsweise das Maß und die Dauer der Expansion vom Flow und/oder der Temperatur des Ausatemluftstroms und/oder einer definierten Fraktion des Ausatemluftstroms abhängig sind.

## Claims

1. A device for the fractional isolation of constituents of exhaled air by changing their state of aggregation by means of the Joule-Thomson effect, which occurs during the expansion of pressurized gas, containing
a flow channel (1) through which the exhaled air can be passed,
a pressure vessel (2) including at least one expansion nozzle (3) connected to said pressure vessel (2) and containing a pressurized gas or gas mixture by means of which, when it expands into the flow channel (1), the temperature of the exhaled air passed through said flow channel (1) can be lowered to a temperature suitable for condensing the constituents of the exhaled air flow which are to be fractionated and/or for freezing them out in the form of frozen particles (4) in the exhaled air flow,
a collection device (5) for receiving the liquid and/or frozen particles (4) carried along in the exhaled air flow, wherein said collection device (5) is provided downstream of the expansion nozzle (3).

2. The device according to claim 1, **characterized in that**
the flow channel (1) has a geometry by means of which flow conditions can be induced in the flow channel (1) which cause the liquid and/or frozen particles (4) carried along in the exhaled air flow to directly separate from the exhaled air flow in the collection device (5).

3. The device according to claim 1 or 2, **characterized in that**
at least one expansion nozzle (3) is provided in the flow channel (1), preferably in the longitudinal central axis of said flow channel (1).

4. The device according to claim 1 or 2, **characterized in that**
at least one expansion nozzle (3) is provided in the wall of the flow channel (1), preferably as an annular nozzle (6).

5. The device according to any one of claims 1 to 4, **characterized in that**
a flow controller (7) is provided, which can be regulated and controlled and by means of which the flow of the expanding gas or gas mixture through the expansion nozzle (3) can be adjusted and adapted to the flow rate of the exhaled air in the flow channel (1).

6. The device according to any one of claims 1 to 5, **characterized in that**
the flow channel (1) has a curve (8), preferably ranging from 45 to 90 degrees.

7. The device according to claim 6, **characterized in that**
the collection device (5) is provided directly in the curve (8) of the flow channel (1).

8. The device according to any one of claims 1 to 7, **characterized in that**
the collection device (5) is provided in the flow channel (1) opposite the inlet opening (9), seen in the direction of flow.

9. The device according to any one of claims 1 to 8, **characterized in that**
the collection device (5) comprises a temperature-modifying element (10) by means of which the temperature of the collection device (5) can be adjusted, controlled and/or regulated.

10. The device according to any one of claims 1 to 9, **characterized in that**
the collection device (5) is provided with pipes and/or sliding surfaces (13) by means of which the liquid and/or frozen particles (4) can be transferred into or onto the collection device (5).

11. The device according to any one of claims 1 to 10, **characterized in that**
a layer (16) the temperature of which can be modified is provided on or in the wall of the flow channel (1).

12. The device according to any one of claims 1 to 11, **characterized in that**
at least one temperature sensor (17) for measuring the temperature of the exhaled air flow is provided in and/or on the wall of the flow channel (1) and/or directly in said flow channel (1).

13. A method for the fractional isolation of constituents of exhaled air by changing the state of aggregation of said constituents by means of the Joule-Thomson effect
where an exhaled air flow passed through a flow channel (1), by expanding a pressurized gas or gas mixture contained in a pressure vessel (2) into said exhaled air flow passed through said flow channel (1), is cooled to a temperature at which the constituents of the exhaled air which are to be fractionated condense so as to form liquid particles (4) and/or freeze out in the form of frozen particles (4) in the exhaled air flow,
the constituents which have been fractionated so as to form liquid and/or frozen particles (4) are carried along in the exhaled air flow and separated in a collection device (5).

14. The method according to claim 13, **characterized in that**
the geometry of the flow channel (1) induces flow conditions in said flow channel (1) which cause the liquid and/or frozen particles (4) to be directly separated in the collection device (5).

15. The method according to either of claims 13 or 14, **characterized in that**
the collection device (5) is cooled to ≤ 0°C while the constituents are fractionated by condensing them and/or freezing them out in the exhaled air flow and separating them in or on said collection device (5).

16. The method according to any one of claims 13 to 15, **characterized in that**
the temperature of a layer (16) the temperature of which can be modified and which is provided in the wall of the flow channel (1) is modified, said layer preferably being heated, while the constituents are fractionated by condensing them and/or freezing them out in the exhaled air flow and separating them in or on the collection device (5).

17. The method according to any one of claims 13 to 16, **characterized in that**
the duration of the gas expansion, the expansion volume and/or the temperature of the exhaled air flow caused by the expansion of the gas or gas mixture is/are controlled and/or regulated, wherein the extent and duration of the expansion preferably depend on the flow and/or the temperature of the exhaled air flow and/or of a defined fraction of the exhaled air flow.

## Revendications

1. Dispositif pour l'extraction fractionnée de composants de l'air expiré par la modification de leur état d'agrégation au moyen de l'effet Joule-Thomson obtenu par la dilatation de gaz sous pression, comprenant un canal d'écoulement (1) à travers lequel l'air expiré peut être guidé,
un récipient sous pression (2) avec au moins une buse de détente (3) raccordée au récipient sous pression (2), contenant un gaz ou un mélange gazeux sous pression avec lequel, par sa dilatation vers l'intérieur du canal d'écoulement (1), la température de l'air expiré guidé dans le canal d'écoulement (1) peut être abaissée à une température qui est appropriée pour condenser les composants à fractionner contenus dans le flux d'air expiré et/ou pour les geler sous forme de particules (4) gelées dans le flux d'air expiré,
un dispositif de récupération (5) pour la réception des particules (4) liquides et/ou gelées entraînées dans le flux d'air expiré, le dispositif de récupération (5) étant aménagé en aval de la buse de détente (3).

2. Dispositif selon la revendication 1, **caractérisé en ce que**
le canal d'écoulement (1) présente une géométrie avec laquelle il est possible d'induire des conditions d'écoulement dans le canal d'écoulement (1) sur la base desquelles une séparation des particules (4) liquides et/ou gelées entraînées dans le flux d'air expiré s'effectue directement à partir du flux d'air expiré contenu dans le dispositif de récupération (5).

3. Dispositif selon la revendication 1 ou 2, **caractérisé en ce**
**qu'**au moins une buse de détente (3) est aménagée dans le canal d'écoulement (1), de préférence dans l'axe médian longitudinal du canal d'écoulement (1).

4. Dispositif selon la revendication 1 ou 2, **caractérisé en ce**
**qu'**au moins une buse de détente (3) est aménagée dans la paroi du canal d'écoulement (1), de préférence sous forme de buse annulaire (6).

5. Dispositif selon une des revendications 1 à 4, **caractérisé en ce**
**qu'**il est aménagé un régulateur de débit (7) pouvant être régulé et commandé, avec lequel il est possible de régler le débit du gaz ou du mélange gazeux en dilatation à travers la buse de détente (3) et d'adapter ce débit à la vitesse d'écoulement de l'air expiré contenu dans le canal d'écoulement (1).

6. Dispositif selon une des revendications 1 à 5, **caractérisé en ce que**
le canal d'écoulement (1) présente une courbure (8), de préférence de 45 à 90 degrés.

7. Dispositif selon la revendication 6, **caractérisé en ce que**
le dispositif de récupération (5) est aménagé directement dans la courbure (8) du canal d'écoulement (1).

8. Dispositif selon une des revendications 1 à 7, **caractérisé en ce que**,
dans le sens de l'écoulement, le dispositif de récupération (5) est aménagé en face de l'ouverture (9) côté entrée dans le canal d'écoulement (1).

9. Dispositif selon une des revendications 1 à 8, **caractérisé en ce que**
le dispositif de récupération (5) présente un élément d'ajustement de température (10) avec lequel la température du dispositif de récupération (5) peut être réglée, commandée et/ou régulée.

10. Dispositif selon une des revendications 1 à 9, **caractérisé en ce que**
le dispositif de récupération (5) est équipé de conduites et/ou de faces de glissement (13) avec lesquelles les particules (4) liquides et/ou gelées peuvent être conduites dans ou sur le dispositif de récupération (5).

11. Dispositif selon une des revendications 1 à 10, **caractérisé en ce**
**qu'**une couche (16) pouvant être ajustée en température est aménagée sur ou dans la paroi du canal d'écoulement (1).

12. Dispositif selon une des revendications 1 à 11, **caractérisé en ce que**,
dans et/ou sur la paroi du canal d'écoulement (1) et/ou dans le canal d'écoulement (1), au moins un capteur de température (17) est aménagé directement pour la mesure de la température du flux d'air expiré.

13. Procédé d'extraction fractionnée de composants de l'air expiré par la modification de l'état d'agrégation des composants de l'air expiré au moyen de l'effet Joule-Thomson,
dans lequel un flux d'air expiré conduit dans un canal d'écoulement (1) est, par la détente d'un gaz ou mélange gazeux contenu dans un récipient sous pression (2) et mis sous pression dans le flux d'air expiré conduit vers l'intérieur du canal d'écoulement (1), refroidi à une température à laquelle les composants à fractionner de l'air expiré contenu dans le flux d'air expiré se condensent en particules (4) liquides et/ou gèlent sous forme de particules (4) gelées
qui sont entraînées en tant que particules (4) liquides et/ou gelées des composants fractionnés dans le flux d'air expiré et qui sont séparées dans un dispositif de récupération (5).

14. Procédé selon la revendication 13, **caractérisé en ce que**,
du fait de la géométrie du canal d'écoulement (1), des conditions d'écoulement sont induites dans le canal d'écoulement (1) et conduisent à ce que les particules (4) liquides et/ou gelées sont séparées directement dans le dispositif de récupération (5).

15. Procédé selon une des revendications 13 ou 14, **caractérisé en ce que**,
pendant le fractionnement des composants par leur condensation et/ou congélation dans le flux d'air expiré et leur séparation dans ou sur le dispositif de récupération (5), il se produit un refroidissement du dispositif de récupération à ≤ 0°C.

16. Procédé selon une des revendications 13 à 15, **caractérisé en ce que**,
pendant le fractionnement des composants par leur condensation et/ou congélation dans le flux d'air expiré et leur séparation dans ou sur le dispositif de récupération (5), il se produit un ajustement de température, de préférence un chauffage, d'une couche (16) pouvant être ajustée en température et aménagée dans la paroi du canal d'écoulement (1).

17. Procédé selon une des revendications 13 à 16, **caractérisé en ce que**
la durée de la dilatation gazeuse, le volume de dilatation et/ou la température du flux d'air expiré suscitée par la dilatation du gaz ou du mélange gazeux sont commandés et/ou régulés, la grandeur et la durée de la dilatation étant de préférence fonction du flow et/ou de la température du flux d'air expiré et/ou d'une faction définie du flux d'air expiré.
